# EUROPEAN PATENT SPECIFICATION

(11) **EP 3 173 846 B1**
(45) Date of publication and mention of the grant of the patent: **04.10.2023**
(21) Application number: 16197626.1
(22) Date of filing: 07.11.2016
(51) Int. Cl.: G02B 27/00, A61B 1/002, G02B 13/00, G02B 23/24

(54) **OPTICAL RELAY SYSTEM**
OPTISCHES RELAISSYSTEM
SYSTÈME DE RELAIS OPTIQUE

(30) Priority: 05.11.2015 US 201514933676; 24.08.2016 DE 102016115738
(43) Date of publication of application: 31.05.2017
(73) Proprietor: Karl Storz SE & Co. KG, 78532 Tuttlingen (DE); Karl Storz Imaging, Inc., Goleta, CA 93117 (US)
(72) Inventor: Duckett III, George E., Goleta, CA 93117 (US); Vogel, Walter, 78532 Tuttlingen (DE)
(74) Representative: Wimmer, Stephan

(56) References cited:
- WO-A1-2016/114081
- US-A- 2 936 673
- US-A- 4 621 910
- US-A- 4 783 154
- US-A- 4 993 817
- US-A- 5 461 509

## Description

The invention relates to an optical relay system. Relay lenses, such as rod lenses, are often used in endoscopes to relay the image from the objective. A relay lens can include a "relay pair" that includes two rod lenses, and multiple relay lenses can be used to extend the distance from the objective to the image. That is, the length of the shaft of an endoscope can be increased by using relay lenses. The image relayed by the relay lenses can be viewed through an eyepiece at the proximal end of the endoscope. Usually, a camera can be coupled to the eyepiece to capture the relayed image.

Conventional relay lenses contribute large amounts of astigmatism and field curvature to the image. These aberrations have traditionally been corrected in the objective optical elements. However, such correction of the aberrations solely in the objective elements leads to designs that are highly sensitive to tilt and decenter errors caused by manufacturing tolerances. For an endoscope with a large number of relays, the aberrations caused by the stack-up of these tilt and decenter errors in manufacturing tolerances can be too severe to correct solely in the objective elements. In addition, burdening the objective elements with excessive aberration correcting properties can increase the required surface curvatures and/or the optical work done by the objective elements. This can further increase tolerance sensitivity of the system.

In GB 1 534 541, a relay lens system with a plurality of relay lens pairs is described, wherein chromatic aberration is corrected within each single relay lens pair.

In US 5,097,359, a configuration of a relay lens for an endoscope is described.

In US 5,142,410, relay lens systems are described, wherein single relay lens pairs are corrected. In US 5,933,275, a relay lens system comprising a plurality of relay lens units is described. At least one relay lens unit allows longitudinal chromatic aberration to remain in an undercorrected tendency and at least another relay lens unit allows longitudinal chromatic aberration to remain in an overcorrected tendency. The relay lens system is configured to correct longitudinal chromatic aberration in the relay lens system as a whole by canceling the undercorrected longitudinal chromatic aberration and the overcorrected longitudinal chromatic aberration with each other.

In US 4,993,817, a relay system 15 for an endoscope 5 is disclosed, the relay system 15 transmitting an image in a first image plane 12 to a second image plane 17 (column 2, lines 29 through 35). The relay system 15 comprises a plurality of cemented five-element assemblies 20 arranged in pairs (column 2, lines 45 through 49). Cemented five-element assemblies 20 are identical and symmetric from end to end (column 2, lines 57 and 58). Each cemented five-element assembly 20 comprises a central rod-like element 22, first and second negative elements 25 separated by and cemented to the central element 22, and first and second positive end elements 27 separated by the central and negative elements and cemented to the negative elements (column 2, lines 58 through 63).

US 2,936,673 refers to polarizing microscopes comprising a polariser in the illumination light path upstream an object and an analyser in the imaging optical path downstream the object. Depolarisation caused by oblique refracting surfaces slightly rotating the plane of polarisation and causing an incomplete extinction and a reduced contrast is compensated for by a no-power or substantially no-power meniscus lens.

It is an object of the present invention to provide an improved relay lens system.

This object is solved by the subject-matter of the independent claim.

The foregoing and other objects are at least partially achieved by provision of various embodiments of the present invention. Aberrations in one or more relay lenses are at least partially compensated for by introducing at least one afocal meniscus lens between an image plane and at least one of the relay lenses. Image plane refers to a plane containing an image formed by an element of an optical system. Example elements include, for example, an objective lens and relay lens, such as a rod lens relay (e.g., Hopkins rod-lens system).

In particular, a lens or another optical facility is afocal if the lens or other optical facility produces no substantial net convergence or divergence of collimated light. In particular, a lens or another optical facility is afocal if the focal length of the lens or other optical facility is infinite or, at least, much greater (in particular by one, two or more orders of magnitude) than the focal length of other optical facilities in the same system; or in other words, if the optical power of the lens or other optical facility is zero or the absolute value of the optical power of the lens or other optical facility is much smaller than the absolute value of other optical facilities in the same system.

A meniscus lens of a system as described herein may have a small positive optical power for some wavelengths within the spectrum the system is made for and a small negative optical power for other wavelengths within the spectrum the system is made for. The optical power of the meniscus lens may be zero for some wavelengths within the spectrum the system is made for and have a small absolute value for other wavelengths within the spectrum the system is made for. In particular, the absolute value of the optical power of the meniscus lens is less than one tenth or less than one hundredth of the absolute value of the optical power of other parts or of each other part or of the rest of the system throughout the spectrum the system is made for.

According to the present invention, a relay lens system for use in an endoscope is provided. The relay lens system defines an optical path and includes a first imaging facility and a first meniscus lens positioned in the optical path and between an intermediate image plane of the relay lens system and the first imaging facility, wherein the first imaging facility and the first meniscus lens define the optical path, and wherein the first meniscus lens is substantially afocal.

In addition to the features described above, further aspects of the present invention can include one or more of the following features, individually or in combination:
- the first meniscus lens is spaced apart from the first imaging facility;
- the meniscus lens is not cemented to a rod lens;
- the relay lens system includes a second imaging facility and a second meniscus lens; the first imaging facility and the first meniscus lens reside on a first side of the intermediate image plane; the second imaging facility and the second meniscus lens reside on a second side of the intermediate image plane; the first and second sides of the intermediate image plane are opposing sides;
- the first and second meniscus lenses are consecutively arranged along the optical path;
- the first imaging facility has a relay lens pair;
- the second imaging facility has a relay lens pair;
- at least one of the relay lens pairs has a first rod lens and a second rod lens;
- the first meniscus lens has a first surface facing the first imaging facility and an opposing second surface facing the intermediate image plane, the first surface of the first meniscus lens being convex;
- the second meniscus lens has a first surface facing the second imaging facility pair and an opposing second surface facing the intermediate image plane, the first surface of the second meniscus lens being convex;
- the first meniscus lens has a first surface and an opposing second surface; the first surface and the second surface of the first meniscus lens each define a radius and together define a thickness extending between the first surface and the second surface; the ratio of the radius of the second surface of the first meniscus lens to the radius of the first surface of the first meniscus lens is about 0.75; the ratio of the radius of the second surface to the thickness of the first meniscus lens is about 1.35;
- the first meniscus lens has a first surface and an opposing second surface; the first surface and the second surface of the first meniscus lens each define a radius and together define a thickness extending between the first surface and the second surface; the second meniscus lens has a first surface and an opposing second surface; the first surface and the second surface of the second meniscus lens each define a radius and together define a thickness extending between the first surface and the second surface; the ratio of the radius of the second surface of the first meniscus lens to the radius of the first surface of the first meniscus lens is about 0.75; the ratio of the radius of the second surface to the thickness of the first meniscus lens is about 1.35; the ratio of the radius of the second surface of the second meniscus lens to the radius of the first surface of the second meniscus lens is about 0.75; the ratio of the radius of the second surface to the thickness of the second meniscus lens is about 1.35;
- an index of refraction of the first meniscus lens is between about 1.7 and about 1.9.
- the first meniscus lens is a doublet having a first lens proximal to the intermediate image plane and a second lens distal from the intermediate image plane;
- the first lens has a ratio of its radius to a total thickness of the doublet of about 1.55:1 and the second lens has a radius to doublet thickness ratio of about 1.2:1;
- the doublet comprises a first lens having an Abbe number of about 47 and a second lens having an Abbe number of about 24;
- the optical system has a Hopkins rod lens system; the Hopkins rod lens system including the first imaging facility;
- the optical system has a Hopkins rod lens system, the Hopkins rod lens system including the first and second imaging facilities; and
- the first lens is a plano-convex lens and the second lens is a plano-concave lens.

An advantage of the present invention is that the afocal meniscus lenses can be used with existing rod lens systems to improve the image quality. Menisci and relay lenses can be mixed and matched for optimum correction of aberrations in the relay lenses. Different designs of afocal meniscus lenses will produce different aberration compensation and correction results.

Other objects, features, and advantages will be apparent from the following detailed description of embodiments of the present invention taken in conjunction with the accompanying drawings. For example, although rod lens relays are shown in the drawings, the present invention includes embodiments utilizing afocal meniscus lens to correct for aberrations in other types of optical relay systems (e.g., a conventional biconvex lens relay system).

A relay lens system for relaying an image from a distal end of the relay lens system to a proximal end of the relay lens system comprises a plurality of imaging facilities, each of the plurality of imaging facilities comprising one or more lenses, wherein each imaging facility of the plurality of imaging facilities relays a real intermediate image located distal to the imaging facility to a further real intermediate image proximal to the imaging facility, wherein the plurality of imaging facilities includes a plurality of first imaging facilities and one second imaging facility, wherein each imaging facility of the plurality of first imaging facilities provides chromatic aberration, wherein the chromatic aberration of the plurality of first imaging facilities is corrected by the second imaging facility, and wherein the second imaging facility is situated between the first imaging facilities.

In particular, the relay lens system is provided and configured for an endoscope. In particular, the relay lens system is provided and configured for relaying a real intermediate image produced by an objective of an endoscope near the distal end of the endoscope and at the distal end of the relay lens system, via a plurality of further real intermediate images, to a last, most proximate real intermediate image at the proximal end of the relay lens system and close to the proximal end of the endoscope. For this purpose, the imaging facilities of the relay lens system are disposed consecutively. The last real intermediate image can be viewed by human eye directly via an eyepiece or captured by a camera or imaged by a further objective or another imaging facility, into the light sensitive layer of an image sensor or into the light sensitive layers of a number of image sensors.

Throughout this specification, an imaging facility is an imaging facility imaging a first real intermediate image situated distal to the imaging facility to a second real intermediate image proximal to the imaging facility without any further real intermediate image between the first real intermediate image and the second real intermediate image. Sometimes, an imaging facility is referred to as an inversion because each imaging facility causes an inversion of the intermediate image in a way that the real intermediate image proximal to the imaging facility is, with respect to the real intermediate image distal to the imaging facility, inverted.

In particular, the first imaging facilities are equal to each other, i.e., apart from differences caused by manufacturing tolerance, cannot be distinguished. In particular, the chromatic aberration of each first imaging facility comprises a longitudinal chromatic aberration and/or a transverse chromatic aberration and/or a chromatic magnification difference. With respect to this chromatic aberration, the first imaging facility is undercorrected, i.e., the chromatic aberration produced by one component of the first imaging facility is not corrected or only partially corrected by another component of the first imaging facility.

In particular, the second imaging facility is overcorrected in so far as not only the chromatic aberration of a component of the second imaging facility but also the chromatic aberration of the first imaging facilities is corrected. For this purpose, the second imaging facility comprises, for example, a correction facility partially or completely correcting the chromatic aberration of the first imaging facilities and of the other components of the second imaging facility. Thus, the second imaging facility alone exhibits chromatic aberration opposite to the chromatic aberration of the first imaging facilities and partially or to a large extent or completely compensating the chromatic aberration of the first imaging facilities. Thereby, in particular, the entire relay lens system is corrected at two or three wavelengths.

In particular, the second imaging facility is disposed at the center between the first imaging facilities. In particular, the relay lens system comprises an even number (for example two, four, six or eight) of first imaging facilities, wherein one half of the first imaging facilities each is disposed distal and proximal to the second imaging facility. In this case, the first imaging facilities are, in particular, disposed symmetrical to the second imaging facility.

In particular, the relay lens system comprises an optical axis which is the optical axis of all imaging facilities. In particular, the first imaging facilities are disposed with reflection symmetry to a symmetry plane orthogonal to the optical axis of the second imaging facility or to the optical axis of the relay lens system.

In a relay lens system as described herein, the second imaging facility can be mirror symmetric with respect to a symmetry plane orthogonal to the optical axis of the second imaging facility.

In a relay lens system as described herein, each first imaging facility comprises, in particular, merely two identical lenses.

In particular, each first imaging facility comprises only two identical rod lenses.

In a relay lens system as described herein, each first imaging facility can comprise two rod lenses, wherein each rod lens is reflected symmetric with respect to a plane of symmetry orthogonal to the optical axis of the first imaging facility.

In a relay lens system as described herein, each first imaging facility comprises, in particular, merely one optically transparent material.

In a relay lens system as described herein, each first imaging facility can be completely uncorrected with respect to chromatic aberration.

In a relay lens system as described herein, the second imaging facility can comprise two rod lenses and a correction facility between the two rod lenses, the correction facility correcting chromatic aberration of the first imaging facilities and of the rod lenses of the second imaging facility.

The two rod lenses of the second imaging facility can be equal. In particular, the two rod lenses of the second imaging facility comprise merely one optically transparent material. In particular, the two rod lenses of the second imaging facility can be completely uncorrected with respect to chromatic aberration.

The correction facility can be provided and configured for correcting one or several different kinds of chromatic aberrations of the first imaging facilities and of the rod lenses of the second imaging facility. The correction facility can be spaced apart from both rod lenses of the second imaging facility. As an alternative, the correction facility can be cemented with one rod lens or with both rod lenses of the second imaging facility. In particular, the second imaging facility comprises merely the two rod lenses and the correction facility between the two rod lenses, i.e., the second imaging facility comprises no other optically effective facilities.

Correction of chromatic aberration of a relay lens system in one imaging facility, namely in the second imaging facility, can reduce the manufacturing costs of the relay lens system. In particular, each of the undercorrected or completely uncorrected first imaging facilities can have a particularly simple structure or construction and, hence, particularly low manufacturing costs.

Disposing the overcorrected second imaging facility at the center of the relay lens system can facilitate a particularly effective correction of chromatic aberration and/or of other aberrations.

Configuration of the second imaging facility with a correction facility between two rod lenses can facilitate a particularly simple design and manufacture of the second imaging facility. In particular, each of the two rod lenses of the second imaging facility can be configured without any cemented lens and, hence, can be manufactured in a particularly simple way.

In a relay lens system as described herein, each of the two rod lenses of the second imaging facility is reflection symmetric with respect to a symmetry plane orthogonal to the optical axis of the second imaging facility.

In a relay lens system as described herein, the correction facility can comprise a reflection symmetric group of a number of cemented lenses.

In a relay lens system as described herein, the correction facility can be reflection symmetric with respect to a symmetry plane orthogonal to the optical axis of the second imaging facility.

A reflection symmetric arrangement of the rod lenses of the second imaging facility and/or of the correction facility of the second imaging facility and/or of part of the correction facility and/or of the entire second imaging facility can reduce the probability of an error during manufacture of the relay lens system.

In a relay lens system as described herein, the correction facility can comprise two similar groups disposed reflection symmetrically wherein each group comprises a plurality of cemented lenses.

In a relay lens system as described herein, the two similar groups disposed reflection symmetrically can be disposed at two plane surfaces of a plate, wherein the plane surfaces are averted from each other.

As an alternative, the two similar groups disposed reflection symmetrically can be spaced apart from each other.

In a relay lens system as described herein, each first imaging facility can comprise two rod lenses or consist of two rod lenses, wherein the two rod lenses of each first imaging facility are similar to the rod lenses of the second imaging facility.

In particular, the two rod lenses of each first imaging facility are similar to the two rod lenses of the second imaging facility in so far as they provide - apart from differences caused by manufacturing tolerance - identical characteristics and cannot be distinguished.

Using similar rod lenses both for the undercorrected first imaging facilities and for the overcorrected second imaging facility can increase the number of similar parts and reduce the number of different parts and, hence, reduce the manufacturing costs. Furthermore, the risk of a mix-up and, thus, of faulty assemblage or faulty manufacture can be reduced further.

In a relay lens system as described herein, the second imaging facility can comprise a plurality of lenses cemented to each other.

In particular, each lens comprises merely one single optically transparent material and, thus, homogeneous optical characteristics. One or more lenses of the second imaging facility can be rod lenses.

In a relay lens system as described herein, each first imaging facility can include a rod lens or another lens comprising a glass with a refractive index not less than 1.60 and not greater than 1.65 and an Abbe number not less than 48 and not greater than 52 or 54.

Glasses with the mentioned characteristics provide a comparatively high refractive index and, simultaneously, a comparatively low dispersion. Therefore, such glasses produce less chromatic aberration than many other glasses.

In a relay lens system as described herein, each first imaging facility can include a rod lens made of a lead-free material.

In particular, each first imaging facility merely comprises lenses made of a lead-free material or several lead-free materials. In particular, the second imaging facility exclusively comprises lenses made of lead-free materials, too.

In particular, the material or the materials comprise no lead within the limits of manufacturing tolerance. In particular, the material comprises lead at most as an unwanted contamination, wherein the concentration of lead does not or not substantially affect the characteristics of the material.

In a relay lens system as described herein, the ratio of the diameter of a rod lens and the radius of curvature of the surfaces of the rod lens through which surfaces light enters and leaves the rod lenses is not less than 0.3 and not greater than 0.55.

An optical system comprises an objective for producing a real intermediate image, a relay lens system as described herein for producing, from the real intermediate image produced by the objective, a further real intermediate image, and an eyepiece for producing, from the further real intermediate image, a virtual image which can be captured by a camera or by human eye.

An optical system as described herein can comprise one relay lens system or two or more relay lens systems as described herein.

In an optical system as described herein, the objective can comprise one or more aspheric lenses.

An aspheric lens is a lens with a surface through which light enters or leaves the lens and which does not provide the shape of a part of a sphere.

In an optical system as described herein, the aspheric lens or one aspheric lens of a plurality of aspheric lenses of the objective can be a meniscus lens.

In an optical system as described herein, the eyepiece can comprise one or a plurality of aspheric lenses.

In an optical system as described herein, the aspheric lens or one aspheric lens out of a plurality of aspheric lenses of the eyepiece can be a meniscus lens.

An endoscope comprises a relay lens system as described herein.

An endoscope as described herein can comprise one relay lens system or a plurality of relay lens systems.

A plurality of relay lens systems can be disposed consecutively along one single optical axis.

An endoscope as described herein can comprise an objective with one or a plurality of aspheric lenses.

In an endoscope as described herein, the aspheric lens or one aspheric lens out of a plurality of aspheric lenses of the objective can be a meniscus lens.

An endoscope as described herein can comprise an eyepiece with one or more aspheric lenses. The aspheric lens or one aspheric lens out of a plurality of aspheric lenses of the eyepiece can be a meniscus lens.

### Short description of the figures

Embodiments will be described below with reference to the enclosed figures.
Figure 1 shows a schematic representation of an endoscope which is not part of the invention;
Figure 2 shows a schematic representation of an optical system of the endoscope shown in Figure 1;
Figure 3 shows a schematic representation of a second imaging facility of the optical system shown in Figure 2;
Figure 4 shows a schematic representation of a proximal end of the optical system shown in Figure 2;
Figure 5 shows a schematic representation of an alternative optical system of the endoscope shown in Figure 1;
Figure 6 shows a schematic representation of a second imaging facility of the optical system shown in Figure 5;
Figure 7 shows a schematic representation of a further alternative optical system of the endoscope shown in Figure 1;
Figure 8 shows a schematic representation of a second imaging facility of the optical system shown in Figure 7;
Figure 9 shows a schematic elevation view of a first embodiment of the invention;
Figure 10 shows a schematic elevation view of a second embodiment of the invention;
Figure 11 shows a schematic elevation view of a third embodiment of the invention;
Figure 12 shows a schematic elevation view of a fourth embodiment of the invention, with an endoscope 510 shown schematically in outline;
Figure 13 shows a schematic elevation view of a fifth embodiment of the invention;
Figure 14 shows a schematic elevation view of a sixth embodiment of the invention.

### Description of embodiments

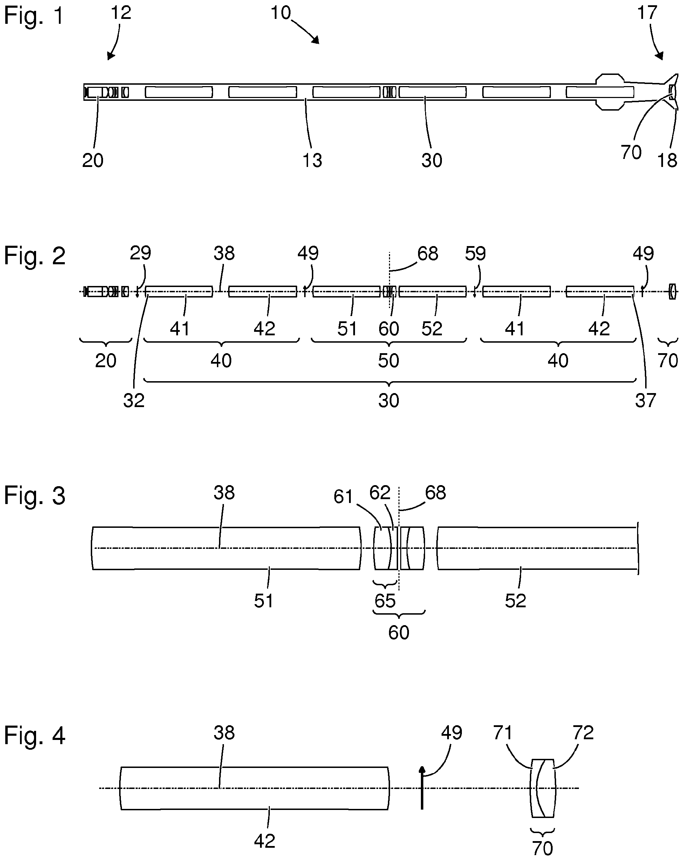

Figure 1 shows a schematic representation of an endoscope, which is not part of the invention, with a distal (in Figure 1: on the left hand side) end 12 and a long straight shaft 13 extending from the distal end 12 to a proximal (in the figures: on the right hand side) end 17 of the endoscope 10. The proximal end 17 of the endoscope 10 is formed by an eyepiece cup 18.

The endoscope 10 includes an objective 20 at the distal end 12, a relay lens system 30 predominantly disposed in the shaft 13 of the endoscope 10, and an eyepiece 70 disposed at the center of a eyepiece cup 18. The objective 20, the relay lens system 30 and the eyepiece 70 form an optical system for transferring or relaying light emanating from an observed object near the distal end 12 of the endoscope 10 to the eye of a user or to a camera which can be coupled to the proximal end 17, in particular to the eyepiece cup 18, of the endoscope 10. The optical system is an observation system in that it facilitates observation of an object disposed distal to the objective 20 through the eyepiece 70.

Figure 2 shows a schematic representation of the optical system comprising the objective 20, the relay lens system 30 and the eyepiece 70 of the endoscope shown in Figure 1. The relay lens system 30 comprises an optical axis 38 which is, concurrently, the optical axis of the objective 20, of the eyepiece 70 and of all optical components of the relay lens system 30.

The relay lens system 30 includes a plurality of consecutively arranged imaging facilities 40, 50. Each imaging facility 40, 50 produces, from a real intermediate image 29, 49, 59 distal to the imaging facility 40, 50, a further real intermediate image 49, 59 proximal to the imaging facility 40, 50. Since each imaging facility 40, 50 inverts the intermediate image 29, 49, 59 (i.e. transposes left and right as well as top and bottom), the imaging facilities 40, 50 are sometimes called inversions.

The relay lens system 30 includes two first imaging facilities 40 disposed symmetrically to the second imaging facility 50. One first imaging facility 40 is disposed distal to the second imaging facility 50, the other first imaging facility 40 is disposed proximal to the second imaging facility 50. The distal end of the distal first imaging facility 40 concurrently forms the distal end 32 of the relay lens system 30. The proximal end of the proximal first imaging facility 40 concurrently forms the proximal end 37 of the relay lens system 30.

The objective 20 reproduces or images an object not shown in Figure 2 distal (in the figures: left) to the objective 20 to a first real intermediate image 29 proximal to the objective 20 and distal to the distal end 30 of the relay lens system 30. The first imaging facility 40 following the objective 20 relays the real intermediate image 29 to a second real intermediate image 49. The second imaging facility 50 relays the second real intermediate image distal to the second imaging facility 50 to a further real intermediate image 59 proximal to the second imaging facility 50. The proximal first imaging facility 40 between the second imaging facility 50 and the eyepiece 70 relays the real intermediate image 59 to a last real intermediate image 49 proximal to the proximal end 37 of the relay lens system 30 and distal to the eyepiece 70. The eyepiece 70 produces, from the most proximate real intermediate image 49, a virtual image which can be directly viewed by a human eye or captured by means of a camera.

Each first imaging facility 40 comprises merely two similar rod lenses 41, 42. Each rod lens 41, 42 is reflection symmetric with respect to a symmetry plane orthogonal to the optical axis 38. Each of the rod lenses 41, 42 of the first imaging facilities 40 comprises a single material, in particular a glass with a refractive index in the interval from 1.60 to 1.65 and an Abbe number in the interval from 48 to 52 or 54. The rod lenses 41, 42 of the first imaging facility 40 are not corrected with respect to chromatic aberration. Since the rod lenses 41, 42 of the first imaging facilities 40 do not comprise cemented lenses, they can be manufactured with low effort and at low costs.

The second imaging facility 50 includes two identical rod lenses 51, 52 and a correction facility 60 between the rod lenses 51, 52. Each of the rod lenses 51, 52 of the second imaging facility 50 comprises a single optically transparent material, in particular a glass with a refractive index in the interval from 1.60 to 1.65 and an Abbe number in the interval from 48 to 52 or 54. The rod lenses 51, 52 of the second imaging facility 50 do not comprise cemented lenses. The rod lenses 51, 52 are equal to each other. Each rod lens 51, 52 of the second imaging facility 50 is reflection symmetric with respect to a symmetry plane orthogonal to the optical axis 38. The rod lenses 51, 52 of the second imaging facility 50 are similar to the rod lenses 41, 42 of the first imaging facility 40.

The first imaging facilities 40 and the rod lenses 51, 52 of the second imaging facility 50 are disposed reflection symmetric with respect to a symmetry plane 68 orthogonal to the optical axis 38. In the example shown in Figure 2, the correction facility 60 is reflection symmetric with respect to the symmetry plane 68, too. Hence, the entire relay lens system 30 is reflection symmetric with respect to the symmetry plane 68.

The correction facility 60 is provided and configured to correct the chromatic aberrations (of one or more kinds, in particular longitudinal chromatic aberration, transverse chromatic aberration, chromatic magnification difference) not only of the rod lenses 51, 52 of the second imaging facility 50 but also of the first imaging facilities 40. In so far, the first imaging facilities 40 are undercorrected and the second imaging facility 50 is overcorrected.

Figure 3 shows a magnified schematic representation of parts of the second imaging facility 50, in particular of the entire distal rod lens 51, the correction facility 60 and part of the proximal rod lens 52.

The correction facility 60 includes four lenses 61, 62 in two similar groups 65 disposed reflection symmetrically with respect to the symmetry plane 68. The lenses 61, 62 of each group are cemented to each other. Both groups 65 are spaced apart from each other. Those surfaces of the lenses 62 which surfaces are facing each other and through which surfaces light enters and leaves the lenses 62 are plane and parallel with other.

Figure 4 shows a schematic representation of the eyepiece 70 and of a part, in particular the proximal rod lens 42, of the proximal first imaging facility 40.

The eyepiece 70 includes two lenses 71, 72 cemented to each other and, thus, forming a group. One lens 71 of the eyepiece 70 is an aspheric meniscus lens.

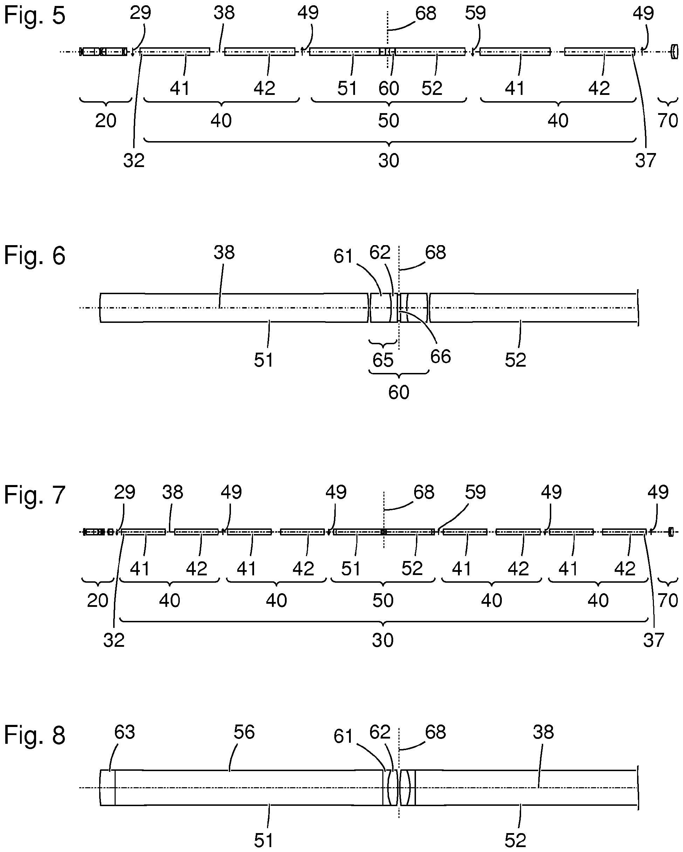

Figure 5 shows a schematic representation of an alternative example of an optical system 20, 30, 70 which is, with respect to a number of features, characteristics and functions, similar to the optical system portrayed with reference to Figs. 2 through 4, and which can be inserted into an endoscope as it is portrayed with reference to Figure 1. Below, in particular features, characteristics and functions of the relay lens system 30 of the optical system 20, 30, 70 shown in Figure 5 differing from those of the relay lens system of the optical system portrayed with reference to Figs. 2 through 4 are described.

The relay lens system 30 of the optical system 20, 30, 70 shown in Figure 5 includes two first imaging facilities 40 and a second imaging facility 50 between the two first imaging facilities 40. The second imaging facility 50 includes a correction facility 60 disposed between two similar rod lenses 51, 52 and differing from the correction facility portrayed with reference to Figure 2.

Figure 6 shows a schematic representation of part of the second imaging facility 50, in particular of the distal rod lens 51, the correction facility 60 and part of the proximal rod lens 52. Similar to the correction facility portrayed with reference to Figure 3, the correction facility 60 includes four lenses 61, 62 in two similar groups 65 disposed reflection symmetrically with reference to a symmetry plane 68 orthogonal to the optical axis 38. Other than the correction facility portrayed with reference to Figure 3, the two groups 65 are joined to two plane surfaces of a plate 66 which surfaces are averted from each other. In so far, the correction facility 60 is formed by four lenses 61, 62, pairs of which are similar and disposed in reflection symmetry, and a plate 66 with plane surfaces in a mechanical unit, wherein the mechanical unit forms one single bigger group.

Figure 7 shows a schematic representation of a further optical system 20, 30, 70 which is, with respect to a number of features and characteristics, similar to the optical system portrayed with reference to Figs. 2 through 6, and which can be used in an endoscope as portrayed with reference to Figure 1. Below, in particular features, characteristics and functions of the relay lens system 30 of the optical system 20, 30, 70 shown in Figure 7 which differ from those of the relay lens system portrayed with reference to Figs. 2 through 6 are described.

In particular, the relay lens system 30 of the optical system 20, 30, 70 shown in Figure 7 differs from the relay lens systems portrayed with reference to Figs. 2 through 6 in the number of the first, undercorrected imaging facilities 40. The relay lens system 30 shown in Figure 7 comprises four first imaging facilities 40. Two first imaging facilities 40 are disposed distal to the second imaging facility 50, i.e. between the objective 20 and the second imaging facility 50. Two first imaging facilities 40 are disposed proximal to the second imaging facility 50, i.e. between the second imaging facility 50 and the eyepiece 70.

Furthermore, the relay lens system 30 of the optical system 20, 30, 70 shown in Figure 7 differs from the relay lens systems portrayed with reference to Figs. 2 through 6 with regard to the configuration of the second, overcorrected imaging facility 50. The second imaging facility 50 includes two similar rod lenses 51, 52 disposed reflection symmetrically with reference to the symmetry plane 68 orthogonal to the optical axis 38 of the relay lens system 30. The entire relay lens system is reflection symmetric with respect to the symmetry plane 68.

Similar to the relay lens systems portrayed with reference to Figs. 2 through 6, in the relay lens system 30 shown in Figure 7, the first imaging facilities are undercorrected, namely uncorrected, and the second imaging facility 50 is overcorrected. The second imaging facility 50 corrects chromatic aberrations of at least one kind (in particular longitudinal chromatic aberration) of all first imaging facilities 40, whereby the entire relay lens system 30 is corrected at least with respect to this kind of chromatic aberration.

Figure 8 shows a magnified schematic representation of part of the second imaging facility 50 of the relay lens system 30 shown in Figure 7, in particular the distal rod lens 51 and part of the proximal rod lens 52.

The rod lenses 51, 52 are similar to each other and are disposed reflection symmetrically to the symmetry plane 68 orthogonal to the optical axis 38. Only the distal rod lens 51 is described below.

In the example shown in Figure 8, the distal rod lens 51 includes a plate 56, i.e. an optically transparent body with two parallel and plane surfaces through which light enters and leaves the body. A first lens 61 is cemented to the proximal surface of the plate 56 through which light leaves the plate 56. A second lens 62 is cemented to the first lens 61 and forms a proximal surface of the distal rod lens 51 through which surface light leaves the rod lens 51. A third lens 63 is cemented to the distal surface of the plate 56 through which surface light enters the plate 56. The third lens 63 forms the surface of the distal rod lens 51 through which light enters the distal rod lens 51.

The plate 56 and the lenses 61, 62, 63 comprise different optically transparent materials, in particular glasses, with different refractive indices and different Abbe numbers. The radii of curvature of all interfaces, the thickness values, the refractive indices and the Abbe numbers of the materials of the plate 56 and of the lenses 61, 62, 63 are selected such that the resulting chromatic aberration of the second imaging facility 50 corrects or compensates the chromatic aberrations of the first imaging facilities 40.

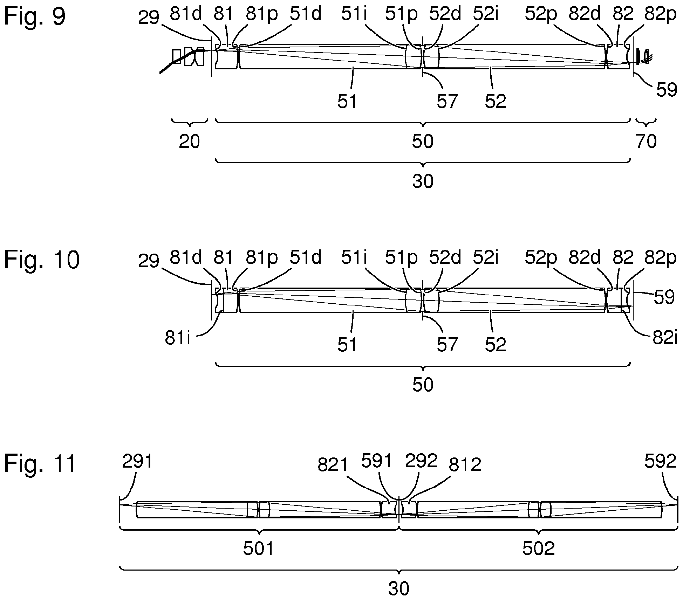

Figure 9 shows a first embodiment of an optical system 20, 30, 70 according to the invention, defining an optical path and including a relay lens system 30 comprising a relay lens, or imaging facility 50, an objective lens 20 at the distal end of the relay lens system 30, and an eyepiece 70 at the proximal end of the relay lens system 30. The imaging facility 50 forming the relay lens system 30 includes a first rod lens 51 and a second rod lens 52. In the embodiment shown in Figure 9, the rod lenses 51, 52 are doublets, which each consist of two simple lenses with shared surfaces 51i, 52i. In other embodiments, however, rod lenses that consist of only a single lens, or compound rod lenses comprising any number of simple lenses, are used. Between the rod lenses 51, 52 in Figure 9 is a stop 57. The stop 57 is located in the afocal space of the imaging facility 50, in which the rays of the image transmitted through the optical system 20, 50, 70 undergo no substantial net convergence or divergence. In some embodiments, the stop 57 can be an aperture stop. As used herein, "stop" refers to openings or structures that limit ray bundles (e.g., an aperture stop). An aperture stop is a stop that determines the ray cone angle, or equivalently the brightness, at an image point.

In the embodiment of Figure 9, each rod lens 51, 52 has an image plane 29, 59 associated with it. The respective image planes 29, 59 are on opposite sides of the rod lenses 51, 52. A first meniscus lens 81 is positioned along the optical path between the image plane 29 and the first rod lens 51 and a second meniscus lens 82 is positioned along the optical path between image plane 59 and second rod lens 52.

In the embodiment of Figure 9, both the rod lenses 51, 52 and the meniscus lenses 81, 82 have distal and proximal surfaces 51d, 51p, 52d, 52p, 81d, 81p, and 82d, 82p. Rod lenses 51, 52 also have shared surfaces 51i, 52i between the two simple lenses. Further, both the first and second meniscus lenses 81, 82 are substantially afocal. That is, the first and second meniscus lenses 81, 82 are designed so as to produce no substantial net convergence or divergence of collimated light. The meniscus lenses 81, 82 serve to at least partially compensate for astigmatism and field curvature introduced into the optical system 20, 50, 70 by, for example, the rod lenses 51, 52.

In some embodiments, including the embodiment shown in Figure 9, the first meniscus lens 81 and the second meniscus lens 82 can be identical lenses oriented in opposite directions in the relay lens system 30. Further, the first and second meniscus lenses 81, 82, which are substantially afocal, can be configured to correct aberrations introduced into the system by the rod lenses. These include astigmatism and field curvature.

The ratio of the radii of the curved surfaces for each of the first and second meniscus lenses 81, 82 can be about 0.75 or 3:4. In other embodiments, this ratio can be different and can be varied alongside the other characteristics of the lens, such as its thickness, index of refraction, Abbe number, etc. The characteristics of the meniscus lenses 81, 82 can be selected so that the meniscus lenses 81, 82 are afocal. It is notable that, because the meniscus lenses 81, 82 are afocal, it is possible to use the imaging facility 50 to relay an image without the meniscus lenses 81, 82. Likewise, the same meniscus lenses 81, 82 can be used with other rod lens relay designs to compensate for aberrations. The invention thus provides increased flexibility for designers - afocal meniscus lenses of different designs can be mixed and matched with rod lens relays of different designs.

As an alternative to the configuration shown in Figure 9, the optical system 30 can comprise a plurality of relay lenses, or imaging facilities 50 arranged in consecutive order.

In an embodiment similar to that shown in Figure 9, the elements of the optical system 20, 30, 70 can have the following characteristics:

| Surface | Radius (mm) | Distance to Next Surface or Thickness (mm) | Index of Refraction | Abbe Number | Outer Diameter (mm) |
|---|---|---|---|---|---|
| Objective | Infinity | 0.6185 | - | - | - |
| First meniscus, distal | -3.2446 | 2.4046 | 1.788 | 47.3 | 1.8 |
| First meniscus, proximal | -4.3062 | 0.1 | - | - | 2.79 |
| First rod lens, proximal | 8.0175 | 19.2 | 1.603 | 38.0 | 2.79 |
| First rod lens, shared | 5.4772 | 1.8 | 1.547 | 53.6 | 2.79 |
| First rod lens, distal | -8.0832 | 0.077 | - | - | 2.79 |
| STOP | Infinity | 0.077 | - | - | 2.79 |
| Second rod lens, distal | 8.0832 | 1.8 | 1.547 | 53.6 | 2.79 |
| Second rod lens, shared | -5.4772 | 19.2 | 1.603 | 38.0 | 2.79 |
| Second rod lens, proximal | -8.0175 | 0.1 | - | - | 2.79 |
| Second meniscus, distal | 4.3062 | 2.4046 | 1.788 | 47.3 | 2.79 |
| Second meniscus, proximal | 3.2446 | 0.6185 | - | - | 1.8 |
| Image | Infinity | - | - | - | - |

In the optical system having the characteristics shown in the foregoing chart, the objective is at the distal end of the relay lens system formed by the first meniscus lens, the first rod lens, the stop, the second rod lens and the second meniscus lens, and each successive surface is the next surface in the proximal direction until the proximal-most image plane is reached. The distances and length measurements are in millimeters.

In the optical system having the characteristics shown in the foregoing chart, the first meniscus lens' distal surface is concave, has a radius of 3.2446 millimeters, an outer diameter of 1.8 millimeters, and is located 0.6185 millimeter from the objective. The thickness of the first meniscus lens is 2.4046 millimeters. The proximal-most surface of the first meniscus lens has a radius of 4.3062 millimeters and an outer diameter of 2.79 millimeters. The first meniscus lens has an index of refraction of 1.788 and an Abbe number of 47.3. The plano-bevel on the distal concave surface of the first meniscus lens is 1.8 millimeters.

The first meniscus lens is 0.1 millimeter from the first rod lens of the relay lens system, which has two elements. The distal-most surface of the distal-most element of the first rod lens has a radius of 8.0175 millimeters and is 19.2 millimeters thick. The surface shared by the two elements of the rod lens has a radius of 5.4772 millimeters and is separated from the distal-most surface of the first rod lens by 1.8 millimeters. The proximal most surface of the first rod lens has a radius of 8.0832 millimeters. The distal-most element of the first rod lens has an index of refraction of 1.603 and an Abbe number of 38.0, while the proximal-most element of the rod lens 201 has an index of refraction of 1.547 and an Abbe number of 53.6.

In the optical system having the characteristics shown in the foregoing chart, the stop in the center of the relay lens system is 0.077 millimeter from both the first and the second rod lenses. The second rod lens is a mirror image of the first rod lens. Thus, the second rod lens has a first surface with a radius of 8.0832 millimeters, a second surface with a radius of 5.4772 millimeters at a distance of 1.8 millimeters away, and a third, proximal-most surface with a radius of 8.0175 millimeters located 19.2 millimeters away. The indices of refraction and Abbe numbers of the elements of the second rod lens also correspond to those of the first rod lens.

In the optical system having the characteristics shown in the foregoing chart, the second meniscus lens is located 0.1 millimeter from the proximal-most surface of the second rod lens. The distal-most surface of the second meniscus lens has a radius of 4.3062 millimeters and the proximal-most surface has a radius of 3.2446 millimeters. The second meniscus lens has a thickness of 2.4046 millimeters, an index of refraction of 1.788, and an Abbe number of 47.3. The image plane is located 0.6185 millimeter from the second meniscus lens.

Figure 10 shows part of a second embodiment of the invention in which the optical system includes an objective lens (not shown), a relay lens system 30 comprising a relay lens, or imaging facility 50 that includes two rod lenses 51, 52, and an eyepiece (not shown). The rod lenses 51, 52 are doublets, in that they each include two simple lenses with a shared surface. Between the rod lenses 51, 52 is stop 57. A first meniscus lens 81 is disposed between image plane 29 and rod lens 51 and a second meniscus lens 82 is disposed between image plane 59 and rod lens 52.

Figure 10 shows each of the first and second meniscus lenses 81, 82 are doublets comprising two simple lenses, as opposed to the single-lensed meniscus lenses of Figure 9. First meniscus lens 81 comprises a plano-concave lens 81a and a plano-convex lens 81b. These lenses are cemented together or otherwise joined in some embodiments, such that they have a distal concave surface 81d, a shared surface 81i, and a proximal convex surface 81p. Likewise, second meniscus lens 82 comprises a plano-concave lens 82b and a plano-convex lens 82a, with distal convex surface 82d, shared surface 82i, and proximal concave surface 82p. The first and second meniscus lenses 81, 82 are substantially afocal lenses. They are designed so as to produce no substantial net convergence or divergence of collimated light.

The first meniscus lens 81 and the second meniscus lens 82 can be identical doublet lenses, but oriented in opposite directions in the relay lens system. As in the first embodiment shown in Figure 9, the first and second meniscus lenses 81, 82 of the embodiment shown in Figure 10 are substantially afocal and configured to correct aberrations introduced into the system by the rod lenses, including astigmatism and field curvature.

The ratio of the radii of the curved surfaces of the first and second meniscus lenses 81, 82 can be about 0.75 or 3:4. In other embodiments, this ratio can be different and can be varied alongside the other characteristics of the lens, such as its thickness, index of refraction, Abbe number, etc. This embodiment shows a design for the afocal meniscus elements including doublets. Numerous other optical designs are possible for use in the present invention, so long as they meet the general requirements of being afocal and a meniscus lens. The meniscus lenses according to the invention can be compound lenses that include more than two lenses, as well.

As an alternative to the configuration described with reference to Figure 10, an optical system can comprise a plurality of relay lenses, or imaging facilities 50 arranged in consecutive order.

In an embodiment similar to that shown in Figure 10, the optical elements can have the following characteristics:

| Surface | Radius (mm) | Distance to Next Surface or Thickness (mm) | Index of Refraction | Abbe Number | Outer Diameter (mm) |
|---|---|---|---|---|---|
| Objective | Infinity | 0.7354 | - | - | - |
| First meniscus, concave | -2.2414 | 0.5 | 1.846 | 23.7 | 1.8 |
| First meniscus, shared | Infinity | 1.384 | 1.788 | 47.3 | 2.79 |
| First meniscus, convex | -2.9286 | 0.1 | - | - | 2.79 |
| First rod lens, proximal | 8.0175 | 19.2 | 1.603 | 38.0 | 2.79 |
| First rod lens, shared | 5.4772 | 1.8 | 1.547 | 53.6 | 2.79 |
| First Rod lens, distal | -8.0832 | 0.077 | - | - | 2.79 |
| STOP | Infinity | 0.077 | - | - | 2.79 |
| Second rod lens, distal | 8.0832 | 1.8 | 1.547 | 53.6 | 2.79 |
| Second rod lens, shared | -5.4772 | 19.2 | 1.603 | 38.0 | 2.79 |
| Second rod lens, proximal | -8.0175 | 0.1 | - | - | 2.79 |
| Second meniscus, convex | 2.9286 | 1.384 | 1.788 | 47.3 | 2.79 |
| Second meniscus, shared | Infinity | 0.5 | 1.846 | 23.7 | 2.79 |
| Second meniscus, concave | 2.2414 | 0.7354 | - | - | 1.8 |
| Image | Infinity | - | - | - | - |

In the optical system having the characteristics shown in the foregoing chart, the objective is at the distal end of the relay lens system formed by the first meniscus lens, the first rod lens, the stop, the second rod lens and the second meniscus lens and each successive surface is the next surface in the proximal direction until the image plane is reached. The distances and length measurements are in millimeters.

The distal surface of the first meniscus lens is located 0.7354 millimeter from the objective of the relay. The distal-most, concave surface of the first meniscus lens has a radius of 2.2414 millimeters. The thickness of the plano-concave lens of the first meniscus lens is 0.5 millimeter. The plano-concave lens has an index of refraction of 1.846 and an Abbe number of 23.7. The thickness of the plano-convex lens of the first meniscus lens is 1.384 millimeters. The convex surface of the meniscus doublet has a radius of 2.9286 millimeters. The plano-convex lens has an index of refraction of 1.788 and an Abbe number of 47.3.

The first meniscus lens is 0.1 millimeter from the distal surface of the first rod lens. The distal-most surface of the distal-most element of the first rod lens has a radius of 8.0175 millimeters and the element is 19.2 millimeters thick. The surface shared by the two elements of the rod lens has a radius of 5.4772 millimeters and is separated from the proximal-most surface of the rod lens by 1.8 millimeters. The proximal most surface of the first rod lens has a radius of 8.0832 millimeters. The distal-most element of the rod lens has an index of refraction of 1.603 and an Abbe number of 38.0, while the proximal-most element of the rod lens has an index of refraction of 1.547 and an Abbe number of 53.6.

The stop in the center of the imaging facility is 0.077 millimeter from both the first and second rod lenses. The second rod lens is a mirror image of the first rod lens. Thus, the second rod lens has a first surface with a radius of 8.0832 millimeters, a second surface with a radius of 5.4772 millimeters at a distance of 1.8 millimeters away, and a third, proximal-most surface with a radius of 8.0175 millimeters located 19.2 millimeters away. The indices of refraction and Abbe numbers of the elements of the second rod lens also correspond to those of the first rod lens. The second meniscus lens is located 0.1 millimeter from the proximal-most surface of the second rod lens. The distal-most, convex surface of the second meniscus lens, has a radius of 2.9286 millimeters. The plano-convex lens has a thickness of 1.384 millimeters, an index of refraction of 1.788, and an Abbe number of 47.3. The concave surface of the plano-concave lens has a radius of 2.2414 millimeters, and the plano-concave lens has a thickness of 0.5 millimeter, an index of refraction of 1.846, and an Abbe number of 23.7. The image plane is located 0.7354 millimeter from the second meniscus lens.

Figure 11 shows part of a third embodiment of the invention in which the optical system includes an objective lens (not shown), a relay lens system 30 comprising two relay lenses, or imaging facilities 501, 502, and an eyepiece (not shown). Each of the imaging facilities501, 502 is substantially identical to the imaging facility 50 shown in Figure 9, except for the positioning of the afocal meniscus lenses. A first afocal meniscus lens 821 is disposed between image plane 591 and imaging facility 501. A second afocal meniscus lens 812 is disposed between image plane 292 and imaging facility 502. No afocal meniscus lenses are disposed between image plane 291 and imaging facility 501 or between image plane 592 and imaging facility 502. In the example shown in Figure 11, image planes 292, 591are the same image plane. The afocal meniscus lenses 821, 812 may be substantially identical to the afocal meniscus lenses described in the embodiment shown in Figure 9. Alternatively, the afocal meniscus lenses may be doublets, as described in the embodiment shown in Figure 10.

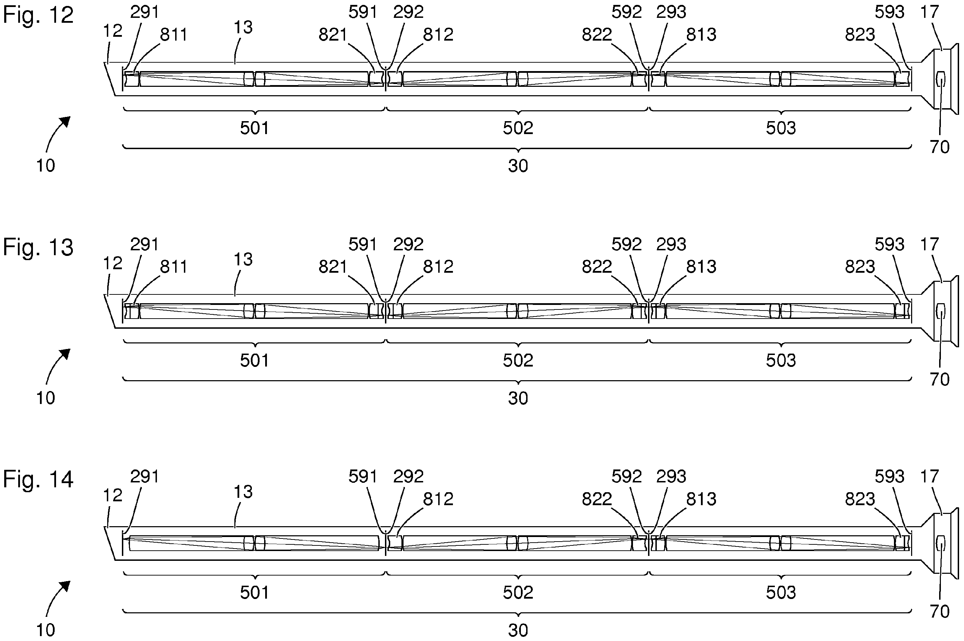

Figure 12 shows a fourth embodiment of an endoscope 10 with an optical system 30, 70 according to the invention that has an objective (not shown), a relay lens system 30 comprising three relay lenses, or imaging facilities 501, 502, 503, and an eyepiece 70. Each of the imaging facilities 501, 502, 503 includes two afocal meniscus lenses 811, 812, 813, 821, 822, 823 disposed between the corresponding image planes 291, 292, 293, 591, 592, 593 and the imaging facilities 501, 502, 503. All of these optical elements and/or components are disposed in the shaft 13 of the endoscope 10. Each of the imaging facilities 501, 502, 503 in Figure 12 is substantially identical to the imaging facility shown in Figure 9. In the example shown in Figure 11, image planes 292, 591 are the same image plane, and image planes 293, 592 are the same image plane.

It is typical and well within the understanding of those of ordinary skill in the art that the endoscope 10 of Figure 12 includes additional optical elements for conditioning and transmitting the image from the distal end 12 to the proximal end 17 of the endoscope 10. These often include, for example, a cover glass, objective lens, aperture stops, and field stops. Some embodiments include coupling elements for attaching the endoscope 10 to a camera or other device.

Figure 13 shows a fifth embodiment of an endoscope 10 with an optical system 30, 70 according to the invention that has an objective lens (not shown), a relay lens system 30 comprising three relay lenses, or imaging facilities 501, 502, 503, and an eyepiece 70. The endoscope 10 is similar to endoscope 10 in Figure 12 except that the afocal meniscus lenses 811, 821, 812, 822, 813, 823 associated with each imaging facility 501, 502, 503 are doublets. Each of the imaging facilities 501, 502, 503 in Figure 13 is substantially identical to the imaging facility shown in Figure 10.

Figure 14 shows a sixth embodiment of an endoscope 10 with an optical system 30, 70 according to the invention that has an objective lens (not shown), a relay lens system 30 comprising three relay lenses, or imaging facilities 501, 502, 503, and an eyepiece 70. The endoscope 10 is similar to the endoscopes shown in Figures 12 and 13, respectively, except that the meniscus lenses 811, 821, 812, 822, 813, 823 associated with each imaging facility 501, 502, 503 are of different types. The distal-most imaging facility 501 is substantially identical to first imaging facilities 40 of the relay lens system 30 portrayed with reference to Figures 2, 5, 7, with no afocal meniscus lens. The intermediate imaging facility 502 is similar or substantially similar or identical to the imaging facility 50 of the relay lens system 30 shown in Figure 9, with two afocal meniscus lenses 812, 822 similar to the afocal meniscus lenses 81, 82 portrayed with reference to Figure 9. The proximal-most imaging facility 503 is similar or substantially similar or identical to the imaging facility 50 of the relay lens system 30 shown in Figure 10, with two doublet afocal meniscus lenses 813, 823 similar to the afocal doublet meniscus lenses 81, 82 portrayed with reference to Figure 10.

### List of reference numerals

- 10: endoscope
- 12: distal end of the endoscope 10
- 13: shaft of the endoscope 10
- 17: proximal end of the endoscope 10
- 18: eyepiece cup of the endoscope 10
- 20: objective of the endoscope 10
- 29: real intermediate image provided by the objective 20
- 30: relay lens system of the endoscope 10
- 32: distal end of the relay lens system 30
- 37: proximal end of the relay lens system 30
- 38: optical axis of the relay lens system 30 and its components
- 40: imaging facility of the relay lens system 30
- 41: first rod lens of the imaging facility 40
- 42: second rod lens of the imaging facility 40
- 49: intermediate image provided by the imaging facility 40
- 50: imaging facility of the relay lens system 30
- 51: first rod lens of the imaging facility 50
- 51i: shared surface of the first rod lens 51
- 52: second rod lens of the imaging facility 50
- 52i: shared surface of the second rod lens 52
- 56: plate of the first rod lens 51
- 57: stop of the imaging facility 50
- 59: real intermediate image provided by the imaging facility 50
- 60: correction facility of the imaging facility 50
- 61: first lens of the correction facility 60
- 62: second lens of the correction facility 60
- 63: third lens of the correction facility 60
- 65: group of the correction facility 60, formed by the first lens 61 and the second lens 62
- 66: plate between the two second lenses 62 of the correction facility 60
- 68: plane of symmetry of the correction facility 60
- 70: eyepiece of the endoscope 10
- 71: first lens of the eyepiece 70
- 72: second lens of the eyepiece 70
- 81: first meniscus lens
- 81a: plano-concave lens of the first meniscus lens 81
- 81b: plano-convex lens of the first meniscus lens 81
- 81d: distal concave surface of the first meniscus lens 81
- 81i: shared surface of the first meniscus lens 81
- 81p: proximal convex surface of the first meniscus lens 81
- 82: second meniscus lens
- 82a: plano-concave lens of the second meniscus lens 82
- 82b: plano-convex lens of the second meniscus lens 82
- 82d: distal concave surface of the second meniscus lens 82
- 82i: shared surface of the second meniscus lens 82
- 82p: proximal convex surface of the second meniscus lens 82
- 291: real intermediate image distal to imaging facility 501
- 292: real intermediate image distal to imaging facility 502
- 293: real intermediate image distal to imaging facility 503
- 501: imaging facility
- 502: imaging facility
- 503: imaging facility
- 591: real intermediate image proximal to imaging facility 501
- 592: real intermediate image proximal to imaging facility 502
- 593: real intermediate image proximal to imaging facility 503
- 811: meniscus lens distal to the imaging facility 501
- 812: meniscus lens distal to the imaging facility 502
- 813: meniscus lens distal to the imaging facility 503
- 821: meniscus lens proximal to the imaging facility 501
- 822: meniscus lens proximal to the imaging facility 502
- 823: meniscus lens proximal to the imaging facility 503

## Claims

1. **Relay lens system** (30) for use in an endoscope, the relay lens system defining an optical path, the relay lens system comprising:
a **first imaging facility** (50; 501, 502, 503); and
a **first meniscus lens** (81, 82; 811, 812, 813, 821, 822, 823) positioned in the optical path and between an intermediate image plane (29; 291, 292, 293; 59; 591, 592, 593) of the relay lens system (30) and the first imaging facility (50; 501, 502, 503),
wherein the first imaging facility (50; 501, 502, 503) and the first meniscus lens (81, 82; 811, 812, 813, 821, 822, 823) define the optical path, and
wherein the first **meniscus lens** (81, 82; 811, 812, 813, 821, 822, 823) is substantially **afocal.**

2. Relay lens system (30) according to the preceding claim, wherein the first meniscus lens (81, 82; 811, 812, 813, 821, 822, 823) is spaced apart from the first imaging facility (50; 501, 502, 503).

3. Relay lens system (30) according to one of the preceding claims, further comprising a **second imaging facility** (502) and a **second meniscus lens** (812), the first imaging facility (501) and the first meniscus (821) lens residing on a first side of the intermediate image plane (292), and the second imaging facility (502) and the second meniscus lens (812) residing on a second side of the intermediate image plane (292), wherein the first and second sides of the intermediate image plane (292) are **opposing sides.**

4. Relay lens system (30) according to claim 3, wherein at least one of
the first meniscus lens (81) comprises a **first surface** (81p) **facing the first imaging facility** (50) and an opposing second surface (81d) facing the intermediate image plane (29), the first surface (81p) of the first meniscus lens (81) being **convex,** and
the second meniscus lens comprises a **first surface facing the second imaging facility** and an opposing second surface facing the intermediate image plane, the first surface of the second meniscus lens being **convex.**

5. Relay lens system (30) according to one of the preceding claims, wherein
the first meniscus lens (81) comprises a first surface (81p) and an opposing second surface (81d) and the first surface (81p) and the second surface (81d) of the first meniscus lens (81) each define a radius and together define a thickness extending between the first surface (81p) and the second surface (81d); and
the ratio of the radius of the second surface (81d) of the first meniscus lens (81) to the radius of the first surface (81p) of the first meniscus lens (81) is about 0.75 and the ratio of the radius of the second surface (81d) to the thickness of the first meniscus lens (81) is about 1.35.

6. Relay lens system (30) according to one of the preceding claims, wherein an **index of refraction** of the **first meniscus lens** (81) is between about **1.7** and about **1.9.**

7. Relay lens system (30) according to one of the preceding claims, wherein the first meniscus lens (81) is a doublet having a first lens (81b) and a second lens (81a), wherein the **first lens** (81b) has a **ratio of its radius to a total thickness** of the doublet of about **1.55:1** and the **second lens** (81a) has a radius to doublet thickness ratio of about **1.2:1.**

8. Relay lens system (30) according to the preceding claim, the first meniscus lens (81) is a doublet having a first lens (81b) and a second lens (81a), wherein the **first lens** (81b) has an **Abbe** number of about **47** and the **second lens** (81a) has an **Abbe** number of about **24.**

9. Relay lens system (30) according to one of the claims 7 or 8, wherein the **first lens** (81b) is a **plano-convex** lens and the **second lens** (81a) is a **plano-concave** lens.

## Patentansprüche

1. **Relaislinsensystem** (30) zur Verwendung in einem Endoskop, wobei das Relaislinsensystem einen optischen Pfad definiert, wobei das Relaislinsensystem umfasst:
eine **erste Abbildungseinrichtung** (50; 501, 502, 503); und
eine **erste Meniskuslinse** (81, 82; 811, 812, 813, 821, 822, 823), die in dem optischen Pfad und zwischen einer Zwischenbildebene (29; 291, 292, 293; 59; 591, 592, 593) des Relaislinsensystems (30) und der ersten Abbildungseinrichtung (50; 501, 502, 503) angeordnet ist,
wobei die erste Abbildungseinrichtung (50; 501, 502, 503) und die erste Meniskuslinse (81, 82; 811, 812, 813, 821, 822, 823) den optischen Pfad definieren, und
wobei die erste **Meniskuslinse** (81, 82; 811, 812, 813, 821, 822, 823) im Wesentlichen **afokal** ist.

2. Relaislinsensystem (30) gemäß dem vorangehenden Anspruch, bei dem die erste Meniskuslinse (81, 82; 811, 812, 813, 821, 822, 823) von der ersten Abbildungseinrichtung (50; 501, 502, 503) beabstandet ist.

3. Relaislinsensystem (30) gemäß einem der vorangehenden Ansprüche, ferner mit einer **zweiten Abbildungseinrichtung** (502) und einer **zweiten Meniskuslinse** (812), wobei die erste Abbildungseinrichtung (501) und die erste Meniskuslinse (821) auf einer ersten Seite der Zwischenbildebene (292) liegen und die zweite Abbildungseinrichtung (502) und die zweite Meniskuslinse (812) auf einer zweiten Seite der Zwischenbildebene (292) liegen, wobei die erste und die zweite Seite der Zwischenbildebene (292) einander **gegenüberliegende Seiten** sind.

4. Relaislinsensystem (30) gemäß Anspruch 3, bei dem zumindest entweder
die erste Meniskuslinse (81) eine **der ersten Abbildungseinrichtung** (50) **zugewandte erste Fläche** (81p) und eine der Zwischenbildebene (29) zugewandte gegenüberliegende zweite Fläche (81d) aufweist, wobei die erste Fläche (81p) der ersten Meniskuslinse (81) **konvex** ist, und
die zweite Meniskuslinse eine **der zweiten Abbildungseinrichtung zugewandte erste Fläche** und eine der Zwischenbildebene zugewandte gegenüberliegende zweite Fläche aufweist, wobei die erste Fläche der zweiten Meniskuslinse konvex ist.

5. Relaislinsensystem (30) gemäß einem der vorangehenden Ansprüche, bei dem
die erste Meniskuslinse (81) eine erste Oberfläche (81p) und eine gegenüberliegende zweite Oberfläche (81d) aufweist, und die erste Oberfläche (81p) und die zweite Oberfläche (81d) der ersten Meniskuslinse (81) jeweils einen Radius definieren und zusammen eine Dicke, die sich zwischen der ersten Oberfläche (81p) und der zweiten Oberfläche (81d) erstreckt, definieren, und
das Verhältnis des Radius der zweiten Oberfläche (81d) der ersten Meniskuslinse (81) zu dem Radius der ersten Oberfläche (81p) der ersten Meniskuslinse (81) etwa 0,75 beträgt und das Verhältnis des Radius der zweiten Oberfläche (81d) zu der Dicke der ersten Meniskuslinse (81) etwa 1,35 beträgt.

6. Relaislinsensystem (30) gemäß einem der vorangehenden Ansprüche, bei dem ein **Brechungsindex** der **ersten Meniskuslinse** (81) zwischen etwa **1,7** und etwa **1,9** liegt.

7. Relaislinsensystem (30) gemäß einem der vorangehenden Ansprüche, bei dem die erste Meniskuslinse (81) ein Dublett mit einer ersten Linse (81b) und einer zweiten Linse (81a) ist, wobei die erste Linse (81b) ein **Verhältnis ihres Radius zu einer Gesamtdicke** des Dubletts von etwa **1,55:1** hat und die **zweite Linse** (81a) ein Verhältnis von Radius zu Dublettdicke von etwa **1,2:1** hat.

8. Relaislinsensystem (30) gemäß dem vorangehenden Anspruch, bei dem die erste Meniskuslinse (81) ein Dublett mit einer ersten Linse (81b) und einer zweiten Linse (81a) ist, wobei die **erste Linse** (81b) eine **Abbe-**Zahl von etwa 47 und die **zweite Linse** (81a) eine **Abbe**-Zahl von etwa **24** hat.

9. Relaislinsensystem (30) gemäß einem der Ansprüche 7 oder 8, bei dem die **erste Linse** (81b) eine **plan-konvexe** Linse und die **zweite Linse** (81a) eine **plan-konkave** Linse ist.

## Revendications

1. **Système de lentilles relais** (30) à utiliser dans un endoscope, le système de lentilles relais définissant un trajet optique, le système de lentilles relais comprenant :
un **premier dispositif d'imagerie** (50 ; 501, 502, 503) ; et
une **première lentille ménisque** (81, 82 ; 811, 812, 813, 821, 822, 823) positionnée dans le trajet optique et entre un plan d'image intermédiaire (29 ; 291, 292, 293 ; 59 ; 591, 592, 593) du système de lentilles relais (30) et le premier dispositif d'imagerie (50 ; 501, 502, 503),
le premier dispositif d'imagerie (50 ; 501, 502, 503) et la première lentille ménisque (81, 82 ; 811, 812, 813, 821, 822, 823) définissent le trajet optique, et
la **première lentille ménisque** (81, 82 ; 811, 812, 813, 821, 822, 823) étant essentiellement afocale.

2. Système de lentilles relais (30) selon la revendication précédente, la première lentille ménisque (81, 82 ; 811, 812, 813, 821, 822, 823) étant espacée du premier dispositif d'imagerie (50 ; 501, 502, 503).

3. Système de lentilles relais (30) selon l'une des revendications précédentes, comprenant en outre un **second dispositif d'imagerie** (502) et une **seconde lentille ménisque** (812), le premier dispositif d'imagerie (501) et la première lentille ménisque (821) se trouvant sur un premier côté du plan d'image intermédiaire (292), et le second dispositif d'imagerie (502) et la seconde lentille ménisque (812) se trouvant sur un second côté du plan d'image intermédiaire (292), les premier et second côtés du plan d'image intermédiaire (292) étant des **côtés opposés.**

4. Système de lentilles relais (30) selon la revendication 3, au moins une des
premières lentilles ménisques (81) comprenant une **première surface** (81p) **orientée vers le premier dispositif d'imagerie** (50) et une seconde surface opposée (81d) orientée vers le plan d'image intermédiaire (29), la première surface (81p) de la première lentille ménisque (81) étant **convexe,** et
la seconde lentille ménisque comprenant une **première surface orientée vers le second dispositif d'imagerie** et une seconde surface opposée orientée vers le plan d'image intermédiaire, la première surface de la seconde lentille ménisque étant **convexe.**

5. Système de lentilles relais (30) selon l'une des revendications précédentes,
la première lentille ménisque (81) comprenant une première surface (81p) et une seconde surface opposée (81d), et la première surface (81p) et la seconde surface (81d) de la première lentille ménisque (81) définissant chacune un rayon et définissant ensemble une épaisseur s'étendant entre la première surface (81p) et la seconde surface (81d) ; et
le rapport entre le rayon de la seconde surface (81d) de la première lentille ménisque (81) et le rayon de la première surface (81p) de la première lentille ménisque (81) étant d'environ 0,75 et le rapport entre le rayon de la seconde surface (81d) et l'épaisseur de la première lentille ménisque (81) étant d'environ 1,35.

6. Système de lentilles relais (30) selon l'une des revendications précédentes, un **indice de réfraction** de la **première lentille ménisque** (81) étant compris entre environ **1,7** et environ **1,9.**

7. Système de lentilles relais (30) selon l'une des revendications précédentes, la première lentille ménisque (81) étant un doublet comportant une première lentille (81b) et une seconde lentille (81a), la **première lentille** (81b) ayant un **rapport entre son rayon et l'épaisseur totale** du doublet d'environ **1,55:1** et la **seconde lentille** (81a) ayant un rapport entre son rayon et l'épaisseur du doublet d'environ **1,2:1.**

8. Système de lentilles relais (30) selon la revendication précédente, la première lentille ménisque (81) étant un doublet comportant une première lentille (81b) et une seconde lentille (81a), la première lentille (81b) ayant un nombre d'**Abbe** d'environ **47** et la seconde lentille (81a) ayant un nombre d'Abbe d'environ **24.**

9. Système de lentilles relais (30) selon l'une des revendications 7 ou 8, la **première lentille** (81b) étant une lentille **plan-convexe** et la seconde lentille (81a) étant une lentille **plan-concave.**
